(19) Europäisches Patentamt
European Patent Office
Office européen des brevets

(11) EP 1 473 026 A1

(12) **EUROPÄISCHE PATENTANMELDUNG**

(43) Veröffentlichungstag:
**03.11.2004 Patentblatt 2004/45**

(51) Int Cl.$^7$: **A61K 7/42**

(21) Anmeldenummer: **04008692.8**

(22) Anmeldetag: **10.04.2004**

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**AL HR LT LV MK**

(30) Priorität: **30.04.2003 DE 10319373**

(71) Anmelder: **Beiersdorf AG**
**20253 Hamburg (DE)**

(72) Erfinder:
- **Müller, Anja**
**23843 Rümpel (DE)**
- **Eitrich, Anja**
**22587 Hamburg (DE)**

(54) **Selbstbräuner enthaltende kosmetische Stifte**

(57) Stiftförmige kosmetische Zubereitungen, die ein oder mehrere selbstbräunende Substanzen und gegebenenfalls weitere kosmetischen und/oder dermatologischen Wirk-, Hilfs- und Zusatzstoffe enthalten.



Printed by Jouve, 75001 PARIS (FR)

**Beschreibung**

Die vorliegende Erfindung betrifft kosmetische Stifte enthaltend mindestens eine selbstbräunende Substanz, sowie deren Verwendung.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Die natürliche Bräunung der Haut durch Sonnenstrahlung wird verursacht durch die Bildung von braunen Farbpigmenten in der Haut. Die Epidermis enthält in ihrer untersten Schicht, der Basalschicht, neben den Basalzellen einzelne pigmentbildende Zellen, die Melanocyten. Durch UV-Licht wird in diesen Zellen die Produktion von Melanin angeregt, das in die Kerantionocycten (Hornzellen) transportiert und dort als braune Hautfarbe sichtbar wird. Diese von der Aminosäure Tyrosin ausgehende Pigmentbildung wird überwiegend durch UVB-Strahlung initiiert und als "indirekte Pigmentierung" bezeichnet. Ihre Entwicklung läuft über mehrere Tage; die so erhaltene Sonnenbräune besteht über einige Wochen.

Bei der "Direkt-Pigmentierung", die mit der Sonnenbestrahlung einsetzt, werden vorwiegend farblose Melanin-Vorstufen durch UVA-Strahlung zu dunkel gefärbten Melanin oxidiert. Da diese Oxidierung reversibel ist, führt sie zu einer nur kurz anhaltenden Hautbräunung.
Die menschliche Haut lässt sich jedoch auch künstlich bräunen. Die künstliche Bräunung der Haut lässt sich beispielsweise äußerlich mit Hilfe von Schminke und oral durch Einnahme von Carotinoiden erzeugen.

Weitaus beliebter jedoch ist die künstliche Bräunung der Haut, welche sich durch Auftragen von sogenannten Selbstbräunern erzielen lässt. Diese Verbindungen weisen als chemisches Strukturmerkmal Keto- bzw. Aldehydgruppen in Nachbarschaft zu Alkoholfunktionen auf. Diese Ketole bzw. Aldole gehören überwiegend zur Substanzklasse der Zucker. Der am häufigsten verwendete Selbstbräuner ist das 1,3-Dihydroxyaceton.

Die Verbindungen können mit den Proteinen und Aminosäuren der Hornschicht der Haut im Sinne einer Maillard-Reaktion umgesetzt werden, wobei über einen noch nicht vollständig aufgeklärten Reaktionsweg Polymerisate entstehen, die der Haut einen bräunlichen Farbton verleihen. Diese Reaktion ist nach etwa 4 bis 6 Stunden abgeschlossen; die so erzielte Bräune ist nicht abwaschbar und wird erst mit der normalen Hautabschuppung entfernt.

Ein Nachteil ist, dass die handelsüblichen Selbstbräunungsprodukte meist als O/W-Lotionen vorliegen und mit der Hand auf der zu bräunenden Körperfläche verteilt werden müssen. Hierbei tritt auch eine Bräunung der Handinnenflächen auf, welche zwangsläufig mit dem Selbstbräunungsprodukt benetzt werden. Nur durch sehr schnelles und gründliches Abwaschen kann dieser Handinnenflächenbräunung entgegengewirkt werden.

Diesem Nachteil des Standes der Technik abzuhelfen, war Aufgabe der vorliegenden Erfindung.

Überraschend gelöst wird die Aufgabe durch stiftförmige kosmetische Zubereitungen, die ein oder mehrere selbstbräunende Substanzen und gegebenenfalls weitere kosmetischen und/oder dermatologischen Wirk-, Hilfs- und Zusatzstoffe enthalten.

Insbesondere die Verwendung von W/O-Stiften stellt eine hervorragende Lösung dar, da hier die Einarbeitung von hydrophilen Selbstbräunern erleichtert ist.

Durch den stiftförmigen Habitus der Zubereitung kann das Kosmetikum gleichmäßig auf der Haut aufgetragen werden, ohne dass hierbei Hautpartien (insbesondere die Handinnenflächen), welche nicht zur Bräunung vorgesehen sind, mit dem Produkt in Kontakt kommen.

Als Selbstbräuner werden erfindungsgemäß vorteilhaft unter anderem eingesetzt:

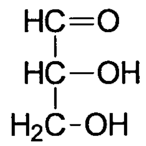

Glycerolaldehyd

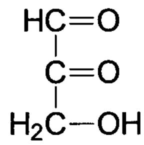

Hydroxymethylglyoxal

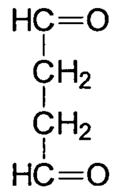

γ-Dialdehyd

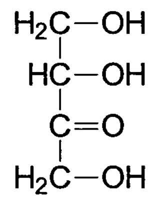

Erythrulose

Ferner ist das 5-Hydroxy-1,4-naphtochinon (Juglon) zu nennen, das aus den Schalen frischer Walnüsse extrahiert wird

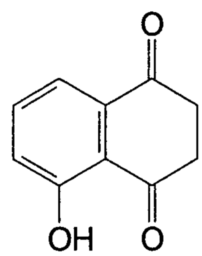

5-Hydroxy-1,4-naphtochinon (Juglon)

sowie das in den Henna-Blättern vorkommende 2-Hydroxy-1,4-naphtochinon (Lawson).

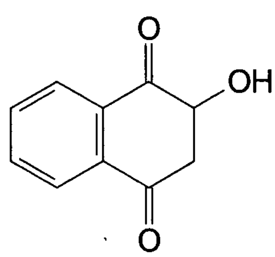

2-Hydroxy-1,4-naphtochinon (Lawson)

Ganz besonders bevorzugt im Sinne der Erfindung ist das 1,3-Dihydroxyaceton (DHA), ein im menschlichen Körper vorkommender dreiwertiger Zucker.

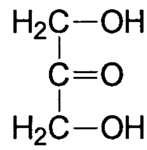

1,3-Dihydroxyaceton (DHA)

Auch 6-Aldo-D-Fructose und Ninhydrin können als erfindungsgemäße Selbstbräuner eingesetzt werden.

Es ist erfindungsgemäß von Vorteil, wenn die erfindungsgemäßen hautbräunenden kosmetischen Zubereitungen eine oder mehrere selbstbräunende Substanzen in einer Konzentration von 0,1 bis 10 Gewichts-% und besonders bevorzugt von 0,5 bis 6 Gewichts-% bezogen auf das Gesamtgewicht der Zubereitung enthalten.

Darüber hinaus können die erfindungsgemäßen Zubereitungen vorteilhaft weitere kosmetische und/oder dermatologische Wirk-, Hilfs- und Zusatzstoffe enthalten.

Die erfindungsgemäßen Zubereitungen können in verschiedenen Formen vorliegen und eingesetzt werden. So können sie z. B. eine Emulsion vom Typ Wasser-in-Öl (W/O) oder eine multiple Emulsion, beispielsweise vom Typ Wasser-in-Öl-in-Wasser (W/O/W) oder eine Pickering-Emulsion darstellen.

Durch die Wahl der Stiftform, ist eine lückenlose, gleichmäßige Applikation auf der Haut möglich, ohne die Handinnenflächen zu verschmutzen.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polyacrylate, Polysaccharide bzw. deren Derivate, z. B. Hyaluronsäure, Xanthangummi, Hydroxypropylmethylcellulose.

Vorteilhaft sind solche kosmetische und dermatologische Zubereitungen, die in der Form eines Sonnenschutzmittels vorliegen. Vorteilhaft enthalten diese mindestens einen UVA-Filter und/oder mindestens einen UVB-Filter und/oder mindestens einen UV-Breitbandfilter und/oder mindestens ein anorganisches Pigment. Es sind dabei alle durch die Positivliste der Kosmetikverordnung der Bundesrepublik Deutschland bzw. entsprechenden Listen der Europäischen Union zugelassenen UV-Lichtschutzfilter erfindungsgemäß vorteilhaft einsetzbar.

Vorteilhaft beträgt die Gesamtmenge der UV-Filtersubstanzen 0,1 Gew.-% bis 30 Gew.-%, vorzugsweise 0,5 bis 20 Gew.-%, insbesondere 1,0 bis 16,0 Gew.-%, bezogen auf das Gesamtgewicht der Zubereitungen, um kosmetische Zubereitungen zur Verfügung zu stellen, welche die Haut vor dem gesamten Bereich der ultravioletten Strahlung schützen. Sie können auch als Sonnenschutzmittel für die Haut dienen.

Es ist auch möglich und gegebenenfalls vorteilhaft, die erfindungsgemäßen Zubereitungen mit anderen Wirkstoffen zu kombinieren.

Die erfindungsgemäßen kosmetischen Zubereitungen können kosmetische Hilfsstoffe enthalten, wie sie üblicherweise in solchen Zubereitungen verwendet werden, z. B. Antioxidantien, Konservierungsmittel, Parfüme, antimikrobielle Wirkstoffe, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Die erfindungsgemäßen Zubereitungen enthalten vorteilhafter Weise Öle und Wachse, welche dazu beitragen den stiftförmigen Zustand zu erreichen und zu erhalten. Vorteilhafte Öle sind hier Caprylic/Capric Triglycerid, Octyldodecanol, Butylen Glycol Dicaprylat/Dicaprat, Bis-Diglyceryl Polyacyladipat-2, Pentaerythrityl Tetraisostearat, Ricinusöl, Lanolinöl, hydriertes Polydecen, Isopropylpalmitat, Cetylpalmitat, Myristyllactat, Avocadoöl, Isostearyl Isostearat, Triisostearin, Oleyl Erucat, Jojobaöl, Dicaprylylcarbonat, Squalan, Diisostearyl Malat, Cyclomethicon, Polyisobuten, Propylencarbonat

Voerteilhafte Wachse sind Cetearylalkohol, Cetylalkohol, Oleylalkohol, Myristyl Myristat, Stearyl Beeswax + Behenyl Beeswax, Bienenwachs, Candelilla Cera, Cera Carnauba, Cera Alba, Cera Microcristallina, C16-40 Alkyl Stearat, C16-36 Alkyl Stearat, C18-38 Alkyl Hydroxystearoyl Stearat, C20-40 Alkyl Stearat, C24-40 Alkyl Stearat, Glycerylbehenat

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, Cholesteryl -und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z. B. Hydroxyfettsäuren, Palmitinsäure, Phytinsäure, Lactoferrin), Hydroxysäuren (z. B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. Linolensäure, Linolsäure, Ölsäure), Folsäure und deren Derivate, Ubichinon und Ubichinol und deren Derivate, Vitamin C und Derivate (z. B. Ascorbylpalmitat, Mg - Ascorbylphosphat, Ascorbylacetat), Tocopherole und Derivate (z. B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, $ZnSO_4$) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,5 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 5 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 5 Gew.-%, bezogen auf das Gesamtgewicht der Formulierung, zu wählen.

Es ist insbesondere vorteilhaft, wenn die kosmetischen und/oder dermatologischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder ß-Alanin.

Erfindungsgemäße Rezepturen, welche z. B. bekannte Antifaltenwirkstoffe wie Flavonglycoside (insbesondere $\alpha$-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Auch können die erfindungsgemäßen Zubereitungen erfindungsgemäß vorteilhaft mit die Haut beruhigenden und pflegenden Substanzen versetzt sein. Hierzu zählen beispielsweise Panthenol, Allantoin, Tannin, Antihistaminika, Antiphlogistika, Glucocorticoide (z.B. Hydrocortison) sowie Pflanzenwirkstoffe wie Azulen und Bisabolol, Glycyrrhizin, Hamamelin und Pflanzenextrakte wie Kamille, aloe vera, Hamazelis, Süßholzwurzel.

Die erfindungsgemäßen Zubereitungen enthalten vorteilhafter Weise Substanzen zur Hautbefeuchtung (engl. Moisturizer). Zu den erfindungsgemäß vorteilhaften Hautbefeuchtungsmitteln zählen unter anderem Polyole wie Glycerin und Sorbit. Auch andere Hautbefeuchtungsmittel wie ethoxylierte Polyole und hydrolysierte Proteine, Komponenten des natürlichen Feuchthaltefaktors der Haut (engl. Natural Moisturizing Factor, NMF) z.B. Harnstoff, Natriumlactat und bestimmte Aminosäuren werden erfindungsgemäß vorteilhaft eingesetzt.

Erfindungsgemäß vorteilhaft beträgt die Konzentration an Hautbefeuchtungsmitteln 1 bis 20 und bevorzugt 3 bis 12 Gew.-% und ganz besonders bevorzugt 5 bis 10 Gew..-%, bezogen auf das Gesamtgewicht der Zubereitung.

Zur Anwendung werden die erfindungsgemäßen kosmetischen Zubereitungen in der für Kosmetika und Dermatika üblichen Weise auf die Haut in ausreichender Menge aufgebracht.

Nicht zuletzt ist die Verwendung von kosmetischen Zubereitungen als Selbstbräuner, Sonnenschutzmittel

und/oder dekoratives Kosmetikum Teil der vorliegenden Erfindung.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Make-up-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente als UV-Filter und/oder zur Stabilisierung der erfindungsgemäßen Zubereitung sind Metalloxide und/oder andere in Wasser schwerlösliche oder unlösliche Metallverbindungen, insbesondere Oxide des Titans ($TiO_2$), Zinks (z. B. ZnO), Eisens (z. B. $Fe_2O_3$), Zirkoniums (z. B. $ZrO_2$) Siliciums (z. B. $SiO_2$) bzw. Silkate (z.B. Talkum), Mangans (z. B. MnO), Aluminiums (z. B. $Al_2O_3$), Cers (z. B. $Ce_2O_3$), Mischoxide der entsprechenden Metalle sowie Abmischungen aus solchen Oxiden, sowie das Sulfat des Bariums ($BaSO_4$).

Im wesentlichen unerheblich für die vorliegende Erfindung ist es, in welcher der gegebenenfalls natürlich vorkommenden Modifikationen die verwendeten Metalloxide vorliegen.

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Ein solches Verfahren, das im folgenden am Beispiel von Titandioxid beschrieben wird, besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste.

Eine weitere vorteilhafte Beschichtung der anorganische Pigmente besteht aus Dimethylpolysiloxan (auch: Dimethicone), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Besonders vorteilhaft sind $TiO_2$-Pigmente, beispielsweise die mit Aluminiumstearat beschichteten, unter der Handelsbezeichnung MT 100 T bei der Firma TAYCA erhältlichen.

Beschriebene beschichtete und unbeschichtete Titandioxide können im Sinne vorliegender Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.
Die erfindungsgemäßen Titandioxide zeichnen sich durch eine Primärpartikelgröße zwischen 10 nm bis 200 nm aus.

| **Handelsname** | **Coating** | **zusätzliche Bestandteile der Vordispersion** | **Hersteller** |
|---|---|---|---|
| MT-100TV | Aluminiumhydroxid<br>Stearinsäure | - | Tayca Corporation |
| MT 100 T | Aluminiumhydroxid<br>Stearinsäure | - | Tayca Corporation |

(fortgesetzt)

| Handelsname | Coating | zusätzliche Bestandteile der Vordispersion | Hersteller |
|---|---|---|---|
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KGaA |
| Eosolex TS | Alumina, Stearinsäure | - | Merck KGaA |
| Titandioxid P25 | None | - | Degussa |
| Titandioxid T805 (Uvinul $TiO_2$) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |

Im Sinne der vorliegenden Erfindung sind besonders bevorzugte Titandioxide das MT-100 Z und MT-100 TV von Tayca Corporation, Eusolex T-2000 und Eusolex TS von Merck und das Titandioxid T 805 von Degussa.

Zinkoxide können im Sinne der vorliegenden Erfindung auch in Form kommerziell erhältlicher öliger oder wässriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| NanoX 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Vorteilhaft im Sinne der vorliegenden Erfindung ist ferner die Verwendung einer Mischung verschiedener Pigmenttypen sowohl innerhalb eines Kristalls, beispielsweise als Eisenmischoxid oder Talkum (Magnesiumsilicat), als auch durch Kombination mehrerer Metalloxidtypen innerhalb einer Zubereitung. Besonders vorteilhaft sind Magnesiumsilicate, beispielsweise die unter der Handelsbezeichnung Talkum Micron bei der Firma Grolmann erhältlichen.

Die Gesamtmenge an einem oder mehreren anorganischen Pigmenten in der fertigen kosmetischen Zubereitung wird vorteilhaft aus dem Bereich 0,1 Gew.-% bis 25 Gew.-% gewählt, vorzugsweise 0,5 Gew.-% bis 18 Gew.-%.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel

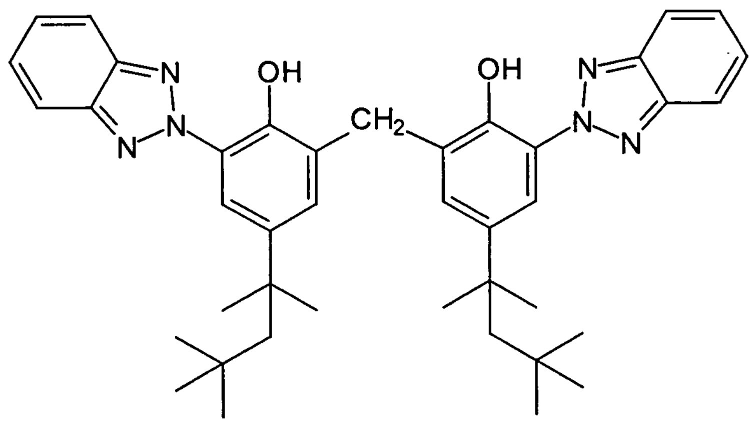

gekennzeichnet ist und unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure

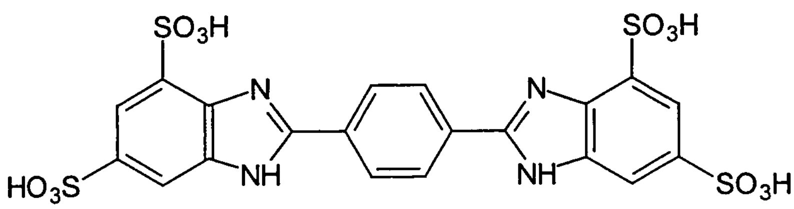

und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz

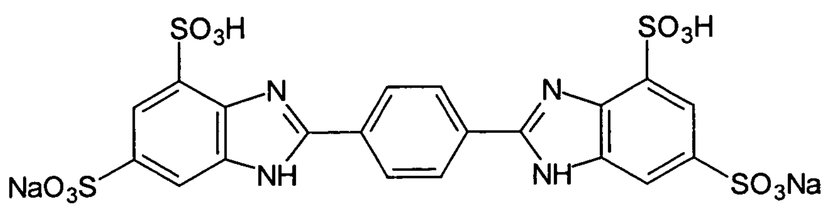

mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

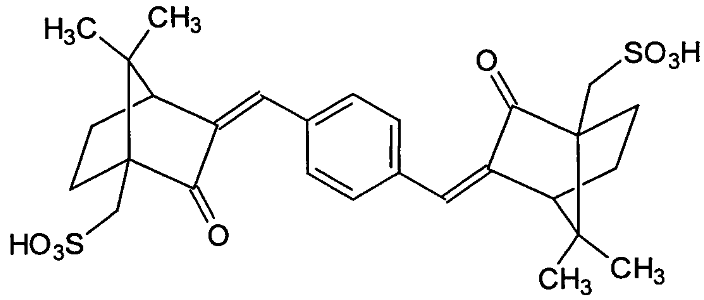

Weitere vorteilhafte UV-A-Filtersubstanzen sind Hydroxybenzophenone, die sich durch die folgende Strukturformel auszeichnen:

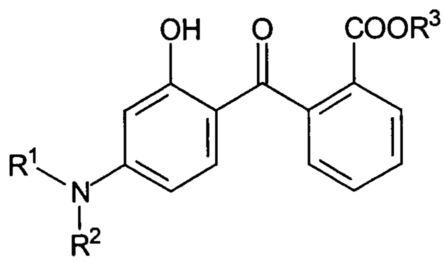

worin

- $R^1$ und $R^2$ unabhängig voneinander Wasserstoff, $C_1$-$C_{20}$-Alkyl, $C_3$-$C_{10}$-Cycloalkyl oder $C_3$-$C_{10}$-Cycloalkenyl bedeuten, wobei die Substituenten $R^1$ und $R^2$ gemeinsam mit dem Stickstoffatom, an das sie gebunden sind, einen 5- oder 6-Ring bilden können und
- $R^3$ einen $C_1$-$C_{20}$-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet:

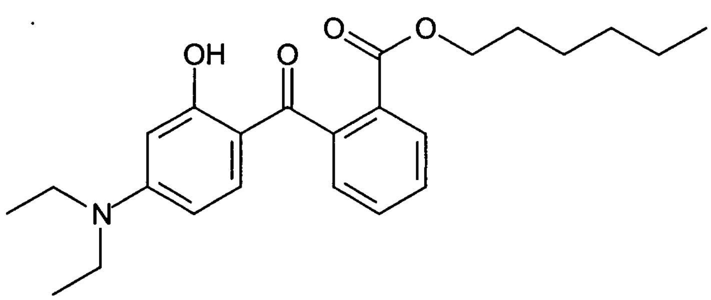

und unter dem Handelsnamen Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur:

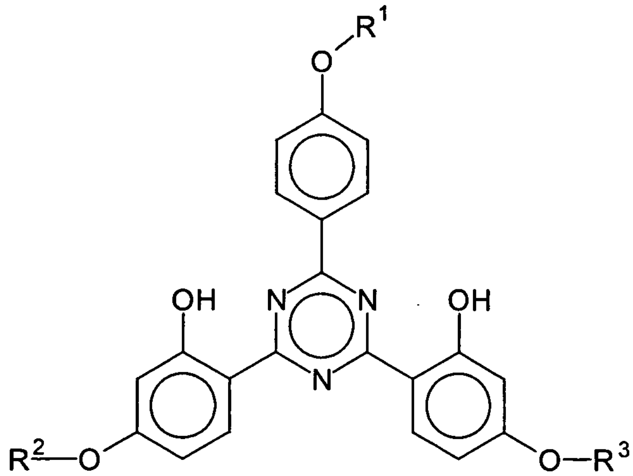

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethylhexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{(4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv

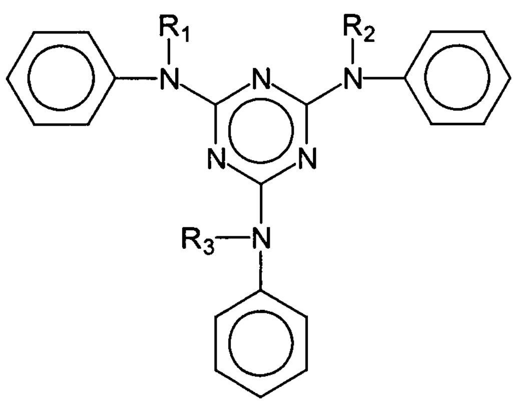

aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel

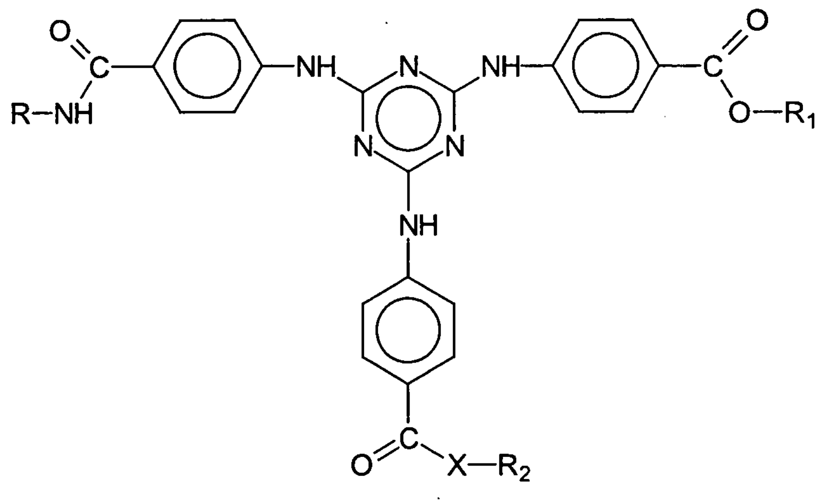

wiedergegeben wird, wobei

- R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,
- X ein Sauerstoffatom oder eine NH-Gruppe darstellt,
- $R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

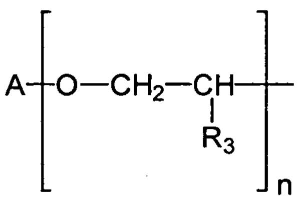

bedeutet, in welcher

- A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
- $R_3$ ein Wasserstoffatom oder eine Methylgruppe darstellt,
- n eine Zahl von 1 bis 10 darstellt,

- $R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

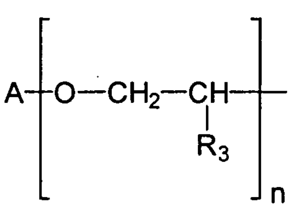

bedeutet, in welcher

| | |
|---|---|
| A | einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen, |
| $R_3$ | ein Wasserstoffatom oder eine Methylgruppe darstellt, |
| n | eine Zahl von 1 bis 10 darstellt, |
| wenn X | ein Sauerstoffatom darstellt. |

Besonders bevorzugte UV-Filtersubstanz im Sinne der vorliegenden Erfindung ist ferner ein unsymmetrisch substituiertes s-Triazin, dessen chemische Struktur durch die Formel

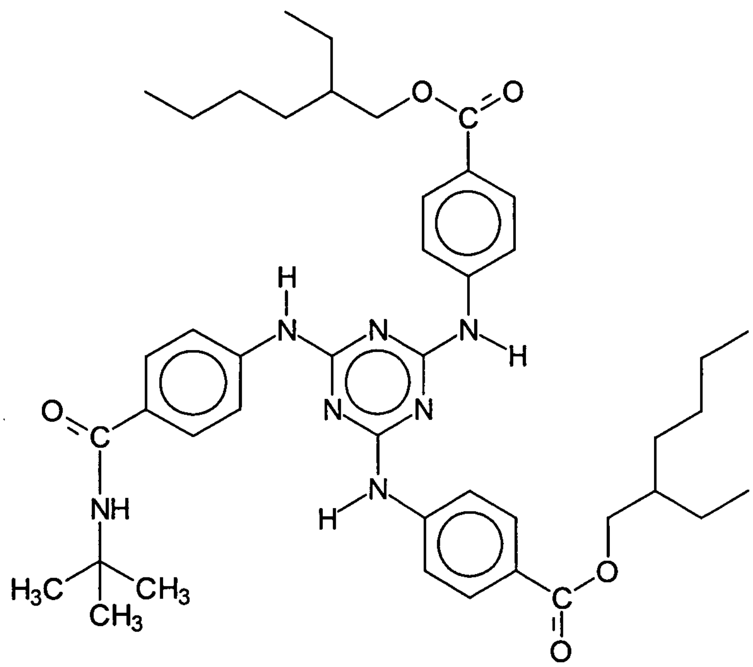

wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel

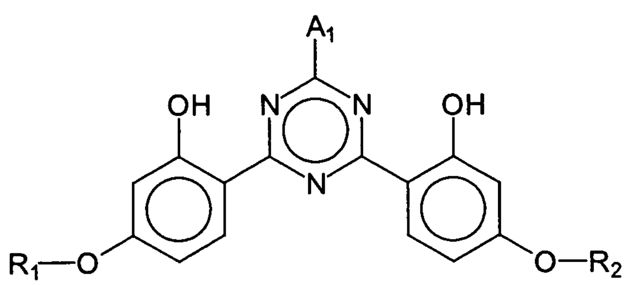

wiedergegeben wird, wobei $R_1$ , $R_2$ und $A_1$ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxypropyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{(4-(1',1',1',3',5',5',5'-Heptamethylsiloxy-2"-methylpropyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylenbis-(6-(2H-benzotriazol-2-yl)-4-(1,1,3,3-tetramethylbutyl)-phenol), welches durch die chemische Strukturformel

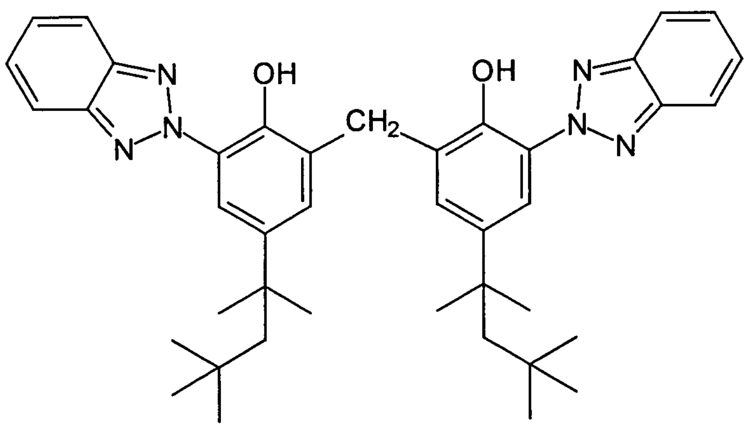

gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2Hbenzotriazol-2-yl)-4-methyl-6-[2-methyl-3-[1, 3, 3,3-tetramethyl-1-[(trimethylsi lyl )oxy]disiloxanyl]propyl]-phenol (CAS-Nr.: 155633-54-8) mit der INCI-Bezeichnung Drometrizole Trisiloxane, welches durch die chemische Strukturformel

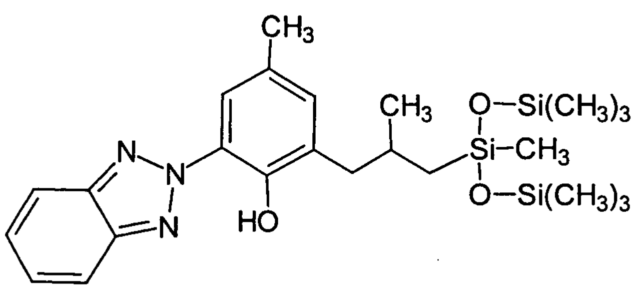

gekennzeichnet ist.

Die UV-B- und/oder Breitband-Filter können öllöslich oder wasserlöslich sein. Vorteilhafte öllösliche UV-B- und/oder Breitband-Filtersubstanzen sind z. B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- sowie an Polymere gebundene UV-Filter.

Vorteilhafte wasserlösliche UV-B- und/oder Breitband-Filtersubstanzen sind z. B.:

- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-hydroxybenzoat (2-Ethylhexylsalicylat, Octylsalicylat, INCI: Octyl Salicylate), 4-Isopropylbenzylsalicylat und Ester der Zimtsäure, vorzugsweise 4-Methoxyzimtsäure (2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan - Copolymer (INCI: Dimethicodiethylbenzalmalonat) welches beispielsweise unter der Handelsbezeichnung Parsol® SLX bei Hoffmann La Roche erhältlich ist.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

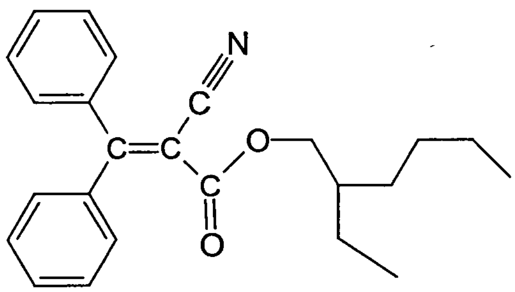

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die erfindungsgemäßen Zubereitungen stellen Abmischungen aus Ölen bzw. öllöslichen Substanzen und Wasser bzw. wasserlöslichen Komponenten dar, die durch den Zusatz von herkömmlichen Emulgatoren und/oder mikronisierten Feststoffpartikeln stabilisiert werden. Die erfindungsgemäßen Zubereitungen stellen stiftförmige Wasser in Öl Emulsionen dar. Besonders vorteilhaft zur Stabilisierung dieser Formulierungen sind W/O-Emulgatoren insbesondere Polyglyceryl-3 Diisostearat, Polyglyceryl-2 Dipolyhydroxystearat, PEG-7 hydriertes Ricinusöl, PEG-45/Dodecyl Glycol Copolymer, Sorbitanisostearat, Pentaerythrylisostearat, Sorbitan Triisostearate, Sucrose Distearat

Erfindungsgemäß können die Zubereitungen anorganische Pigmente mit amphiphilem Charakter, die sowohl in Wasser als auch in Öl dispergierbar sind, enthalten.

Die erfindungsgemäßen Zubereitungen stellen Abmischungen aus Ölen bzw. öllöslichen Substanzen und Wasser bzw. wasserlöslichen Komponenten dar, die durch den Zusatz von mikronisierten Feststoffpartikeln stabilisiert werden und keinen Emulgator im herkömmlichen Sinn enthalten müssen. Eine Erklärungsmöglichkeit für die Stabilität dieser Zubereitungen ist, daß sich die Pigmentpartikel an die Tröpfchen der dispersen Phase anlagern und sozusagen eine mechanische Barriere bilden, die das Koaleszieren, d. h. das Zusammenfließen der Tröpfchen, verhindert.

Die erfindungsgemäßen Zubereitungen sind in jeglicher Hinsicht überaus befriedigende Präparate, die erstaunlicherweise höchst variabel in ihrem Wasser-Fettphasen-Verhältnis sind. Es war insbesondere überraschend, daß die erfindungsgemäßen Stiftzubereitungen einen gegenüber dem Stand der Technik deutlich erhöhten Wassergehalt aufweisen können. Sie zeichnen sich durch exzellente kosmetische Eigenschaften aus und bieten sich daher für den Einsatz in vielen Bereichen der pflegenden und dekorativen Kosmetik an. Für viele Anwendungen ist es insbesondere vorteilhaft, daß die erfindungsgemäßen Zubereitungen frei von Emulgatoren im herkömmlichen Sinne sein können. Die erfindungsgemäßen Zubereitungen sind ferner hervorragende Vehikel für verschiedenste Wirkstoffe.

Vorteilhaft ist es, den mittleren Partikeldurchmesser der verwendeten Pigmente zwischen 1 nm und 200 nm, besonders vorteilhaft zwischen 5 nm und 100 nm zu wählen.

Es ist vorteilhaft im Sinne der vorliegenden Erfindung die Metalloxide aus der Gruppe Titandioxid, Zinkoxid, Eisenoxide bzw. Eisenmischoxide, Siliciumdioxid bzw. Silicate (z. B. Talkum) zu wählen, wobei die Metalloxide sowohl einzeln als auch im Gemisch vorliegen können.

Vorteilhaft im Sinne der vorliegenden Erfindung ist es, zur Stabilisierung der Pickering-Emulsionen unbehandelte, nahezu reine Pigmentpartikel zu verwenden, insbesondere solche, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Pickering-Emulsionen im Sinne der vorliegenden Erfindung werden vorteilhaft durch anorganische Pigmente stabilisiert, die oberflächlich wasserabweisend behandelt ("gecoatet") sind, wobei gleichzeitig der amphiphile Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Es ist ferner vorteilhaft, wenngleich nicht zwingend, die erfindungsgemäßen amphiphilen anorganischen Mikropigmente mit weiteren Pigmenten zu kombinieren, beispielsweise mit Titandioxidpigmenten, die mit Octylsilanol beschichtet sind, und/oder mit Siliciumdioxidpartikeln, die oberflächlich wasserabweisend behandelt sind. Zur Kombination geeignete Siliciumdioxidpartikel sind beispielsweise sphärische Polyalkylsilsesquioxan-Partikel wie sie in der Europäischen Offenlegungsschrift 0 686 391 erwähnt werden. Solche Polyalkylsilsesquioxan-Partikel sind beispielsweise unter den Handelsbezeichnungen Aerosil R972 und Aerosil 200V bei der Firma Degussa erhältlich. Geeignete Titandioxidpartikel sind unter der Handelsbezeichnung T805 ebenfalls bei der Firma Degussa erhältlich.

Als weitere vorteilhafte Pigmente können Bornitride eingesetzt werden, beispielsweise durch die im folgenden aufgelisteten Bornitride:

| Handelsname | erhältlich bei |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN® | Carborundum |
| Wacker-Bornitrid BNP | Wacker-Chemie |

Vorteilhaft ist es, den mittleren Partikeldurchmesser der verwendeten Bornitridpartikel kleiner als 20 μm, besonders vorteilhaft kleiner als 15 μm zu wählen.

Pickering-Emulsionen im Sinne der vorliegenden Erfindung werden ebenfalls vorteilhaft durch Bornitridpartikel stabilisiert, die oberflächlich wasserabweisend behandelt ("gecoatet") sind, wobei gleichzeitig der amphiphile Charakter gebildet werden bzw. erhalten bleiben soll.

Eine vorteilhafte Beschichtung der Bornitridpartikel besteht aus Dimethylpolysiloxan (Dimethicon). Vorteilhaft sind beispielsweise die bei der Firma Carborundum unter der Handelsbezeichnung Tres BN® UHP 1106 erhältlichen, mit Dimethicon behandelten Bornitridpartikel.

Vorteilhaft ist ferner eine Beschichtung der Bornitridpartikel mit Polymethylhydrogensiloxan, einem linearen Polysiloxan, welches auch als Methicone bezeichnet wird. Vorteilhafte, mit Methicone behandelte Bornitridpartikel sind beispielsweise die bei der Firma Carborundum unter der Handelsbezeichnung Tres BN® UHP 1107 erhältlichen.

Es ist ferner vorteilhaft, die erfindungsgemäßen Pickering-Emulsionen durch mikrofeine Polymerpartikel zu stabilisieren.

Vorteilhafte mikrofeine Polymerpartikel sind im Sinne der vorliegenden Erfindung beispielsweise Polycarbonate, Polyether, Polyethylen, Polypropylen, Polyvinylchlorid, Polystyrol, Polyamide, Polyacrylate und dergleichen mehr.

Erfindungsgemäß vorteilhaft sind beispielsweise mikrofeine Polyamid-Partikel, welche unter der Handelsbezeichnung SP-500 bei der Firma TORAY erhältlich sind. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε-laurinlactam) aus ε-Laurinlactam. Vorteilhaft im Sinne der vorliegenden Erfindung sind beispielsweise Orgasol® 1002 (Polyamid 6) und Orgasol® 2002 (Polyamid 12) von der Firma ELF ATO-CHEM.

Weitere vorteilhafte Polymerpartikel sind mikrofeine Polymethacrylate. Solche Partikel sind beispielsweise unter der Handelsbezeichnung POLYTRAP® bei der Firma DOW CHEMICAL erhältlich.

Es ist insbesondere vorteilhaft, wenngleich nicht zwingend, wenn die verwendeten mikrofeinen Polymerpartikel oberflächlich beschichtet sind. Diese Oberflächenbehandlung kann darin bestehen, daß die Pigmente nach an sich bekannten Verfahren mit einer dünnen hydrophilen Schicht versehen werden. Vorteilhafte Beschichtungen bestehen beispielsweise aus $TiO_2$ $ZrO_2$ oder auch weiteren Polymeren, wie beispielsweise Polymethylmethacrylat.

Besonders vorteilhafte mikrofeine Polymerpartikel im Sinne der vorliegenden Erfindung sind ferner nach dem in der US-Patentschrift 4,898,913 beschriebenen Verfahren zur hydrophilen Beschichtung hydrophober Polymerpartikel erhältlich.

Vorteilhaft in ist es, den mittleren Partikeldurchmesser der verwendeten mikrofeinen Polymerpartikel kleiner als 100 µm, besonders vorteilhaft kleiner als 50 µm zu wählen. Dabei ist es im wesentlichen unerheblich, in welcher Form (Plättchen, Stäbchen, Kügelchen etc.) die verwendeten Polymerpartikel vorliegen.

Desweiteren ist es vorteilhaft, die erfindungsgemäßen Pickering-Emulsionen durch modifizierte Polysaccharide zu stabilisieren.

Modifizierte Polysaccharide im Sinne der vorliegenden Erfindung sind beispielsweise durch Umsetzung von Stärke mit mono-, bi- oder polyfunktionellen Reagenzien bzw. Oxidations-Mitteln in weitgehend polymeranalog verlaufenden Reaktionen erhältlich.

Solche Reaktionen basieren im wesentlichen auf Umwandlungen der Hydroxy-Gruppen der Polyglucane durch Veretherung, Veresterung oder selektive Oxidation. Dabei entstehen z. B. sogenannte Stärkeether und Stärkeester der allgemeinen Strukturformel

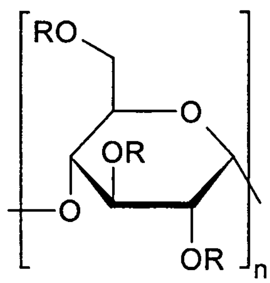

**Strukturformel (I)**

worin R beispielsweise ein Wasserstoff und/oder einen Alkyl- und/oder Aralkylrest (im Fall der Stärkeether) oder ein Wasserstoff und/oder einen organischen und/oder anorganischen Säure-Rest (im Fall der Stärkeester) darstellen kann. Stärkeether und Stärkeester sind vorteilhafte modifizierte Polysaccharide im Sinne der vorliegenden Erfindung.

Besonders vorteilhafte Stärkeether sind z. B. solche, die durch Veretherung von Stärke mit Tetramethylolacetylendiharnstoff erhältlich sind und welche als Amylum non mucilaginosum (nicht quellende Stärke) bezeichnet werden.

Insbesondere vorteilhaft sind ferner Stärkeester und deren Salze, beispielsweise die Natrium- und/oder Aluminiumsalze niedrigsubstituierter Halbester der Stärke, insbesondere Natrium Stärke n-Octenylsuccinat der Strukturformel (I), worin R sich durch die folgende Struktur auszeichnet

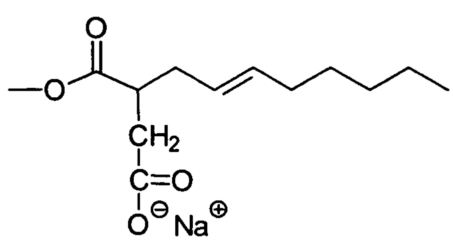

und welches z. B. unter der Handelsbezeichnung Amiogum® 23 bei der Firma CERESTAR erhältlich ist sowie Aluminium Stärke Octenylsuccinate, insbesondere die unter den Handelsbezeichnungen Dry Flo® Elite LL und Dry Flo® PC bei der Firma CERESTAR erhältlichen.

Vorteilhaft ist es, den mittleren Partikeldurchmesser der verwendeten modifizierten Polysaccharide kleiner als 20 µm, besonders vorteilhaft kleiner als 15 µm zu wählen.

Die Liste der genannten modifizierten Polysaccharide, die erfindungsgemäße Pickering-Emulsionen stabilisieren können, soll selbstverständlich nicht limitierend sein. Modifizierte Polysaccharide im Sinne der vorliegenden Erfindung sind auf zahlreichen, an sich bekannten Wegen, sowohl chemischer als auch physikalischer Natur erhältlich.

Die vorstehend genannten amphiphilen Partikel eignen sich hervorragend sowohl zur Stabilisierung von W/O-Pickering-Emulsionen als auch zur Stabilisierung von O/W-Pikkering-Emulsionen. Nachfolgend werden erfindungsgemäße mikrofeine Partikel genannt, welche vorteilhaft insbesondere einen der beiden Emulsionstypen W/O bzw. O/W stabilisieren.

Vorteilhaft in allen vorgenannten Fällen ist es, die Konzentration der erfindungsgemäßen amphiphilen Pigmente größer als 1 Gew.-%, insbesondere zwischen 1 Gew.-% und 30 Gew.-%, besonders vorteilhaft zwischen 2,5 Gew.-% und 7,5 Gew.-%, bezogen auf das Gesamtgewicht der Zubereitungen, zu wählen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| Beispiel 1: | |
|---|---|
| | **Gew.-%** |
| Caprylic/Capric Triglycerid | 12 |
| Octyldodecanol | 7 |
| Bis-Diglyceryl Polyacyladipat-2 | 6 |
| Pentaerythrityl Tetraisostearat | 8 |
| Ricinusöl | 15 |
| Polyglyceryl-3 Diisostearat | 2,5 |
| silanisiertes Titandioxid | 1 |
| Cetearylalkohol | 6 |
| Myristyl Myristat | 2 |
| Bienenwachs | 2 |
| Cera Carnauba | 1,5 |
| Cera Alba | 0,5 |
| Tocopheryl Acetat | 0,5 |
| Tocopherol; Ascorbyl Palmitat | 0,05 |
| Buxus Chinensis | 2 |
| Parfum, BHT | q.s. |
| Preservatives | q.s. |
| DHA | 7 |
| Wasser | ad 100 |
| Magnesium Sulfat | 0,5 |
| Glycerin | 5 |
| Butylenglykol | 1 |

| Beispiel 2: | |
|---|---|
| | **Gew.-%** |
| Caprylic/Capric Triglycerid | 10 |
| Octyldodecanol | 14 |

(fortgesetzt)

| Beispiel 2: | |
|---|---|
| | **Gew.-%** |
| Bis-Diglyceryl Polyacyladipat-2 | 6 |
| Pentaerythrityl Tetraisostearat | 5 |
| Titandioxid Aluminium/Silica gecoatet | 3 |
| Zinkoxid | 1 |
| Myristyl Myristat | 2 |
| Cera Carnauba | 2 |
| Cera Alba | 0,5 |
| C16-40 Alkyl Stearat | 1,5 |
| C20-40 Alkyl Stearat | 10 |
| Butyl Methoxydibenzoylmethan | 1 |
| mikronisiertes Titandioxid (silanisiert) | 1 |
| 4-Methylbenzylidene Campher | 3,6 |
| Ethylhexyl Methoxycinnamat | 3,6 |
| Ethylhexylsalicylat | 2 |
| Tocopheryl Acetat | 1 |
| Parfum, BHT | q.s. |
| Preservatives | q.s. |
| DHA | 3 |
| Wasser | ad 100 |
| NaCl | 1 |
| Butylenglykol | 5 |

| Beispiel 3: | |
|---|---|
| | **Gew.-%** |
| Caprylic/Capric Triglycerid | 6 |
| Octyldodecanol | 8 |
| Bis-Diglyceryl Polyacyladipat-2 | 8 |
| Pentaerythrityl Tetraisostearat | 6 |
| Ricinusöl | 20 |
| Polyglyceryl-3 Diisostearat | 1,8 |
| PEG-45/Dodecyl Glycol Copolymer | 0,9 |
| Cetearylalkohol | 6 |
| Bienenwachs | 4 |
| Cera Carnauba | 2 |
| C16-40 Alkyl Stearat | 1,5 |

(fortgesetzt)

| Beispiel 3: | |
|---|---|
| | **Gew.-%** |
| Butyl Methoxydibenzoylmethan | 1 |
| mikronisiertes Titandioxid (silanisiert) | 0,5 |
| Ethylhexyl Methoxycinnamat | 7,5 |
| Octocrylen | 7,5 |
| Benzophenon-3 | 3,5 |
| Parfum, BHT | q.s. |
| Preservatives | q.s. |
| DHA | 1 |
| Wasser | ad 100 |
| Butylenglykol | 10 |

| Beispiel 4: | |
|---|---|
| | **Gew.-%** |
| Octyldodecanol | 3 |
| Butylen Glycol Dicaprylat/Dicaprat | 12 |
| Bis-Diglyceryl Polyacyladipat-2 | 6 |
| Pentaerythrityl Tetraisostearat | 5 |
| Ricinusöl | 15 |
| Titandioxid Aluminium/Silica gecoatet | 0,5 |
| Zinkoxid | 4 |
| Silica | 0,5 |
| Bienenwachs | 4 |
| Cera Alba | 0,5 |
| C16-40 Alkyl Stearat | 1 |
| Tocopherol; Ascorbyl Palmitat | 0,1 |
| Parfum, BHT | q.s |
| Preservatives | q.s |
| DHA | 1,5 |
| Wasser | ad 100 |
| Glycerin | 10 |

| Beispiel 5: | |
|---|---|
| | **Gew.-%** |
| Lanolinöl | 6 |

(fortgesetzt)

| Beispiel 5: | |
|---|---|
| | **Gew.-%** |
| hydriertes Polydecen | 10 |
| Myristyllactat | 4 |
| Bis-Diglyceryl Polyacyladipat-2 | 4 |
| Pentaerythrityl Tetraisostearat | 2 |
| Avocadoöl | 4 |
| Jojobaöl | 2 |
| PVP/Hexadecen Copolymer | 1 |
| Ricinusöl | 15 |
| Pentaerythrylisostearat | 1,8 |
| PEG-45/Dodecyl Glycol Copolymer | 1 |
| Interferenc Pigment | 6,8 |
| silanisiertes Titandioxid | 4 |
| Silica | 0,5 |
| Cetylalkohol | 1,5 |
| Candelilla Cera | 4,5 |
| Butyl Methoxydibenzoylmethan | 0,2 |
| 4-Methylbenzylidene Campher | 2 |
| Ethylhexyl Methoxycinnamat | 0,5 |
| Octocrylen | 2 |
| Diethylhexyl Butamido Triazon | 1 |
| Tocopheryl Acetat | 1 |
| Parfum, BHT | q.s. |
| Preservatives | q.s. |
| DHA | 0,5 |
| Wasser | ad100 |
| Glycerin | 5 |

| Beispiel 6: | |
|---|---|
| | **Gew.-%** |
| Lanolinöl | 10 |
| Propylencarbonat | 0,12 |
| Myristyllactat | 3 |
| Octyldodecanol | 11,5 |
| Butylen Glycol Dicaprylat/Dicaprat | 3 |
| Bis-Diglyceryl Polyacyladipat-2 | 6 |

(fortgesetzt)

| Beispiel 6: | |
|---|---|
| | **Gew.-%** |
| Avocadoöl | 4 |
| Jojobaöl | 2 |
| Dicaprylylcarbonat | 2 |
| PVP/Hexadecen Copolymer | 3 |
| Ricinusöl | 10 |
| | |
| Disteardimonium Hectorit | 0,5 |
| Titandioxid Aluminium/Silica gecoatet | 3 |
| Candelilla Cera | 4 |
| Ethylhexyl Methoxycinnamat | 2 |
| Bis-Ethylhexyloxyphenol Methoxyphenyl Triazin | 1,5 |
| Diethylhexyl Butamido Triazon | 1,5 |
| Tocopheryl Acetat | 1 |
| Parfum, BHT | q.s. |
| Preservatives | q.s. |
| DHA | 3 |
| Wasser | ad100 |
| Glycerin | 3 |
| Propylenglykol | 2 |

| Beispiel 7: | |
|---|---|
| | **Gew.-%** |
| Lanolinöl | 8 |
| hydriertes Polydecen | 3 |
| Isopropylpalmitat | 4 |
| Octyldodecanol | 3 |
| Bis-Diglyceryl Polyacyladipat-2 | 4 |
| Dicaprylylcarbonat | 2 |
| Squalan | 2 |
| Ricinusöl | 25 |
| Sorbitanisostearat | 2 |
| PEG-45/Dodecyl Glycol Copolymer | 0,9 |
| Interferenc Pigment | 0,6 |
| Cetylalkohol | 1,5 |
| Bienenwachs | 0,6 |

(fortgesetzt)

| Beispiel 7: | |
|---|---|
| | **Gew.-%** |
| Candelilla Cera | 6 |
| Parfum, BHT | q.s. |
| Preservatives | q.s. |
| DHA | 2,5 |
| Wasser | ad 100 |
| Magnesium Sulfat | 0,7 |
| Propylenglykol | 5 |

| Beispiel 8: | |
|---|---|
| | **Gew.-%** |
| hydriertes Polydecen | 3 |
| Isopropylpalmitat | 4 |
| Myristyllactat | 1 |
| Caprylic/Capric Triglycerid | 3 |
| Octyldodecanol | 2 |
| Bis-Diglyceryl Polyacyladipat-2 | 5 |
| Pentaerythrityl Tetraisostearat | 2 |
| Triisostearin | 3 |
| Diisostearyl Malat | 0,7 |
| Ricinusöl | 25 |
| Polyglyceryl-3 Diisostearat | 0,5 |
| Titandioxid Aluminium/Silica gecoatet | 2 |
| Zinkoxid | 0,5 |
| Candelilla Cera | 6,6 |
| C24-40 Alkyl Stearat | 2 |
| Terephthaliden Dicampher | 0,5 |
| Tocopheryl Acetat | 1,5 |
| Parfum, BHT | q.s |
| Preservatives | q.s |
| DHA | 1,5 |
| Wasser | ad100 |
| Citronensäure/NaCitrat | 1 |
| Glycerin | 10 |

| Beispiel 9: | |
|---|---|
| | **Gew.-%** |
| Isopropylpalmitat | 5 |
| Caprylic/Capric Triglycerid | 5 |
| Bis-Diglyceryl Polyacyladipat-2 | 5 |
| Isostearyl Isostearat | 5 |
| Oleyl Erucat | 2 |
| Ricinusöl | 15 |
| Sorbitan Triisostearate | 2 |
| Sucrose Distearat | 1,4 |
| Zinkoxid | 3 |
| Silica | 1 |
| Oleylalkohol | 3 |
| Bienenwachs | 1,9 |
| Candelilla Cera | 2,8 |
| Bis-Ethylhexyloxyphenol Methoxyphenyl Triazin | 0,3 |
| Phenyl Dibenzimidazol Tetrasulfonsäure | 1,5 |
| Phenylbenzmidazol Sulfonsäure | 0,5 |
| Tocopheryl Acetat | 2 |
| Parfum, BHT | q.s. |
| Preservatives | q.s. |
| DHA | 5 |
| Wasser | ad 100 |

| Beispiel 10: | |
|---|---|
| | **Gew.-%** |
| hydriertes Polydecen | 5,5 |
| Cetylpalmitat | 10 |
| Octyldodecanol | 14 |
| Bis-Diglyceryl Polyacyladipat-2 | 2,5 |
| Zinkoxid | 4 |
| Bornitrid | 1 |
| Cetearylalkohol | 2,8 |
| Myristyl Myristat | 3,8 |
| Cera Microcristallina | 22 |
| Candelilla Cera | 0,5 |
| Cera Carnauba | 1,3 |

(fortgesetzt)

| Beispiel 10: | |
|---|---|
| | **Gew.-%** |
| Cera Alba | 1 |
| C20-40 Alkyl Stearat | 2 |
| Butyl Methoxydibenzoylmethan | 1 |
| Ethylhexyl Methoxycinnamat | 7,5 |
| Octocrylen | 7,5 |
| Benzophenon-3 | 3,5 |
| Tocopheryl Acetat | 1 |
| Buxus Chinensis | 1 |
| Butyrospermum Parkii | 1 |
| Parfum, BHT | q.s. |
| Preservatives | q.s. |
| DHA | 0,5 |
| Wasser | ad 100 |
| NaCl | 0,5 |

| Beispiel 11: | |
|---|---|
| | **Gew.-%** |
| Cetylpalmitat | 7 |
| Caprylic/Capric Triglycerid | 18 |
| Octyldodecanol | 22 |
| Polyglyceryl-3 Diisostearat | 3,8 |
| Bornitrid | 1,5 |
| Mica | 0,5 |
| Cetearylalkohol | 2 |
| Myristyl Myristat | 4 |
| Cera Microcristallina | 18 |
| Cera Carnauba | 1,3 |
| Cera Alba | 0,5 |
| C20-40 Alkyl Stearat | 0,5 |
| Buxus Chinensis | 1 |
| Butyrospermum Parkii | 1 |
| Parfum, BHT | q.s. |
| Preservatives | q.s. |
| DHA | 3 |
| Wasser | ad 100 |
| Glycerin | 3 |

| Beispiel 12: | |
|---|---|
| | **Gew.-%** |
| Cyclomethicon | 4 |
| Polyisobuten | 7 |
| Isopropylpalmitat | 5 |
| Octyldodecanol | 7 |
| Titandioxid Aluminium/Silica gecoatet | 3 |
| Zinkoxid | 1 |
| Silica | 0,5 |
| Paraffin | 20 |
| Cera Microcristallina + Paraffinum Liquidum | 16 |
| Cera Microcristallina | 10 |
| synthetischer Bienenwachs | 2 |
| Cera Carnauba | 3,5 |
| Butyl Methoxydibenzoylmethan | 0,5 |
| 4-Methylbenzylidene Campher | 3,6 |
| Ethylhexyl Methoxycinnamat | 3,6 |
| Tocopheryl Acetat | 0,5 |
| Calendula Officinalis + Chamomilla Recutita + Melissa Officinalis | 0,5 |
| Bisabolol | 0,5 |
| Prunus Dulcis | 1 |
| Persea Gratissima | 1 |
| Lanolinalkohol | 0,8 |
| Allantoin | 0,1 |
| Parfum, BHT | q.s |
| Panthenol | 0,1 |
| Preservatives | q.s |
| DHA | 1 |
| Wasser | ad 100 |
| Magnesium Sulfat | 0,5 |
| Propylenglykol | 1 |

| Beispiel 13: | |
|---|---|
| | **Gew.-%** |
| Caprylic/Capric Triglycerid | 8 |
| Octyldodecanol | 8 |
| Bis-Diglyceryl Polyacyladipat-2 | 5 |
| Pentaerythrityl Tetraisostearat | 8 |

(fortgesetzt)

| Beispiel 13: | |
|---|---|
| | **Gew.-%** |
| Polyglyceryl-2 Dipolyhydroxystearat | 1,8 |
| PEG-45/Dodecyl Glycol Copolymer | 0,9 |
| Polyurethan-4 | 1 |
| Cetearylalkohol | 9 |
| Myristyl Myristat | 4 |
| Stearyl Beeswax + Behenyl Beeswax | 6 |
| Cera Carnauba | 2 |
| Cera Alba | 0,5 |
| C16-36 Alkyl Stearat | 10 |
| C18-38 Alkyl Hydroxystearoyl Stearat | 2 |
| C20-40 Alkyl Stearat | 1,5 |
| Butyl Methoxydibenzoylmethan | 1,5 |
| Ethylhexyl Methoxycinnamat | 6 |
| Octocrylen | 6 |
| Benzophenon-3 | 3,5 |
| Buxus Chinensis | 1 |
| Parfum, BHT | q.s. |
| Preservatives | q.s. |
| DHA | 0,5 |
| Wasser | ad100 |
| Citronensäure/NaCitrat | 0,5 |
| Butylenglykol | 5 |

| Beispiel 14: | |
|---|---|
| | **Gew.-%** |
| Octyldodecanol | 3 |
| Butylen Glycol Dicaprylat/Dicaprat | 10 |
| Bis-Diglyceryl Polyacyladipat-2 | 8 |
| Pentaerythrityl Tetraisostearat | 7 |
| PEG-7 hydriertes Ricinusöl | 1,8 |
| PEG-45/Dodecyl Glycol Copolymer | 0,9 |
| Cetearylalkohol | 9 |
| Myristyl Myristat | 3 |
| Stearyl Beeswax + Behenyl Beeswax | 6 |
| Cera Carnauba | 1,5 |

(fortgesetzt)

| Beispiel 14: | |
|---|---|
| | **Gew.-%** |
| Cera Alba | 0,5 |
| C16-36 Alkyl Stearat | 15 |
| C18-38 Alkyl Hydroxystearoyl Stearat | 2 |
| C20-40 Alkyl Stearat | 1,5 |
| Butyl Methoxydibenzoylmethan | 0,5 |
| Octocrylen | 3 |
| Diethylhexyl Butamido Triazon | 2 |
| Tocopheryl Acetat | 1 |
| Buxus Chinensis | 1 |
| Parfum, BHT | q.s. |
| Preservatives | q.s. |
| DHA | 5 |
| Wasser | ad100 |

| Beispiel 15: | |
|---|---|
| | **Gew.-%** |
| Caprylic/Capric Triglycerid | 14 |
| Octyldodecanol | 26 |
| Bis-Diglyceryl Polyacyladipat-2 | 3,8 |
| Polyglyceryl-3 Diisostearat | 1,5 |
| Magnesiumstearat | 0,2 |
| Cera Alba | 0,5 |
| C18-38 Alkyl Hydroxystearoyl Stearat | 20 |
| C20-40 Alkyl Stearat | 0,5 |
| Ethylhexyl Methoxycinnamat | 3 |
| Diethylhexyl Butamido Triazon | 2 |
| Tocopheryl Acetat | 0,5 |
| Bisabolol | 0,5 |
| Butyrospermum Parkii | 1 |
| Parfum, BHT | q.s. |
| Retinyl Palmitat | 0,2 |
| Natrium Ascorbyl Phosphat | 0,2 |
| Panthenol | 3 |
| Preservatives | q.s. |
| DHA | 1 |

(fortgesetzt)

| Beispiel 15: | |
|---|---|
| | **Gew.-%** |
| Wasser | ad 100 |
| Magnesium Sulfat | 0,2 |
| Glycerin | 10 |

| Beispiel 16: | |
|---|---|
| | **Gew.-%** |
| Polyisobuten | 5,5 |
| Caprylic/Capric Triglycerid | 5,6 |
| Octyldodecanol | 3 |
| Mica | 2,5 |
| Zinkstearat | 0,7 |
| Cera Microcristallina + Paraffinum Liquidum | 52 |
| Cera Microcristallina | 2 |
| Butyl Methoxydibenzoylmethan | 1,5 |
| Ethylhexyl Methoxycinnamat | 5 |
| Diethylhexyl Butamido Triazon | 1 |
| Methylen Bis-Benzotriazolyl Tetramethylbutylphenol | 2 |
| Buxus Chinensis | 1,1 |
| Bisabolol | 0,1 |
| Parfum, BHT | q.s |
| Preservatives | q.s |
| DHA | 3 |
| Wasser | ad 100 |

| Beispiel 17: | |
|---|---|
| | **Gew.-%** |
| Polyisobuten | 40 |
| hydriertes Polydecen | 1,8 |
| Myristyllactat | 8 |
| Pentaerythrityl Tetraisostearat | 10 |
| Disteardimonium Hectorit | 1,8 |
| Silica | 1,8 |
| Mica + Cl 77491 | 1 |
| Cl 77891+Mica + Silica | 2 |

(fortgesetzt)

| Beispiel 17: | |
|---|---|
| | **Gew.-%** |
| Butyl Methoxydibenzoylmethan | 0,3 |
| Ethylhexyl Methoxycinnamat | 2,5 |
| Octocrylen | 2,5 |
| Tocopheryl Acetat | 1 |
| Parfum, BHT | q.s. |
| Preservatives | q.s. |
| DHA | 5 |
| Wasser | ad 100 |
| Citronensäure/NaCitrat | 0,5 |

| Beispiel 18: | |
|---|---|
| | **Gew.-%** |
| Lanolinöl | 9 |
| hydriertes Polydecen | 7,3 |
| Isopropylpalmitat | 3 |
| Propylencarbonat | 0,1 |
| Myristyllactat | 5 |
| Octyldodecanol | 8 |
| Bis-Diglyceryl Polyacyladipat-2 | 5 |
| PVP/Hexadecen Copolymer | 1,5 |
| Ricinusöl | 10 |
| Disteardimonium Hectorit | 0,5 |
| Nylon-12 | 0,5 |
| Cl 77891+Mica | 2,1 |
| Mica | 2,5 |
| Cera Microcristallina | 7,5 |
| Candelilla Cera | 6 |
| Cera Carnauba | 2,5 |
| Butyl Methoxydibenzoylmethan | 2 |
| Ethylhexyl Methoxycinnamat | 3 |
| Bis-Ethylhexyloxyphenol Methoxyphenyl Triazin | 1,7 |
| Tocopheryl Acetat | 1 |
| Buxus Chinensis | 2 |
| Persea Gratissima | 3 |
| Lanolinalkohol | 0,9 |

(fortgesetzt)

| Beispiel 18: | |
|---|---|
| | **Gew.-%** |
| Lauryl PCA | 3 |
| Lecithin | 0,5 |
| DHA | 0,5 |
| Wasser | ad 100 |
| Magnesium Sulfat | 0,8 |

| Beispiel 19: | |
|---|---|
| | **Gew.-%** |
| hydriertes Polydecen | 8 |
| Caprylic/Capric Triglycerid | 5 |
| Octyldodecanol | 10 |
| Isostearyl Isostearat | 5 |
| Ricinusöl | 5 |
| Polyglyceryl-3 Diisostearat | 1,8 |
| PEG-45/Dodecyl Glycol Copolymer | 0,9 |
| Myristyl Myristat | 8 |
| C20-40 Alkyl Stearat | 15 |
| Parfum, BHT | q.s. |
| Preservatives | q.s. |
| DHA | 5 |
| Wasser | ad100 |
| Hydroxyethylcellulose | 0,5 |
| Glycerin | 3 |

| Beispiel 20: | |
|---|---|
| | **Gew.-%** |
| Polyisobuten | 10 |
| Propylencarbonat | 5 |
| Octyldodecanol | 10 |
| Butylen Glycol Dicaprylat/Dicaprat | 5 |
| Ricinusöl | 5 |
| Polyglyceryl-3 Diisostearat | 2,5 |
| Glycerylbehenat | 6 |
| Cetylalkohol | 6 |

(fortgesetzt)

| Beispiel 20: | |
|---|---|
| | **Gew.-%** |
| Myristyl Myristat | 5 |
| C16-40 Alkyl Stearat | 6 |
| C18-38 Alkyl Hydroxystearoyl Stearat | 6 |
| Parfum, BHT | q.s |
| Preservatives | q.s |
| DHA | 8 |
| Wasser | ad100 |
| Hydroxyethylcellulose | 0,3 |
| Natrium Corn Starch Octenylsuccinat | 0,3 |
| Magnesium Sulfat | 0,5 |
| Butylenglykol | 3 |

| Beispiel 21: | |
|---|---|
| | **Gew.-%** |
| Cyclomethicon | 5 |
| hydriertes Polydecen | 10 |
| Caprylic/Capric Triglycerid | 10 |
| Butylen Glycol Dicaprylat/Dicaprat | 5 |
| Polyglyceryl-2 Dipolyhydroxystearat | 1,8 |
| PEG-45/Dodecyl Glycol Copolymer | 0,9 |
| Cetearylalkohol | 6 |
| Glycerylbehenat | 6 |
| C24-40 Alkyl Stearat | 6 |
| C18-38 Alkyl Hydroxystearoyl Stearat | 6 |
| Parfum, BHT | q.s. |
| Preservatives | q.s. |
| DHA | 3 |
| Wasser | ad100 |
| Natrium Corn Starch Octenylsuccinat | 0,6 |
| Propylenglykol | 3 |

**Patentansprüche**

**1.** Stiftförmige kosmetische Zubereitungen, die ein oder mehrere selbstbräunende Substanzen und gegebenenfalls weitere kosmetischen und/oder dermatologischen Wirk-, Hilfs- und Zusatzstoffe enthalten.

**2.** Stiftförmige kosmetische Zubereitung nach Anspruch 1, **dadurch gekennzeichnet, dass** sie eine oder mehrere

selbstbräunende Substanzen in einer Konzentration von 0,1 bis 10 Gewichts-% und besonders bevorzugt von 0,5 bis 6 Gewichts-%, jeweils bezogen auf das Gesamtgewicht der Zubereitung enthalten.

**3.** Stiftförmige kosmetische Zubereitung nach mindestens einem der vorhergehenden Ansprüche, **dadurch gekennzeichnet, dass** sie einen Wasseranteil von 0,5 bis 60% insbesondere von 5 bis 40% aufweisen.

**4.** Stiftförmige kosmetische Zubereitung nach mindestens einem der vorhergehenden Ansprüche, **dadurch gekennzeichnet, dass** sie im wesentlichen emulgatorfrei sind, insbesondere einen Gehalt von emulgierenden Substanzen von weniger als 0,3 Gewichts-% aufweisen.

**5.** Stiftförmige kosmetische Zubereitung nach mindestens einem der vorhergehenden Ansprüche, **dadurch gekennzeichnet, dass** sie als selbstbräunende Substanz 1,3-Dihydroxyaceton enthalten.

**6.** Stiftförmige kosmetische Zubereitung nach mindestens einem der vorhergehenden Ansprüche, **dadurch gekennzeichnet, dass** die kosmetische Zubereitung eine Wasser-in-Öl Emulsion ist.

**7.** Stiftförmige kosmetische Zubereitung nach mindestens einem der vorhergehenden Ansprüche, **dadurch gekennzeichnet, dass** der Gehalt an anorganischen Pigmenten in der fertigen kosmetischen Zubereitung im dem Bereich 0,1 Gew.-% bis 25 Gew.-% liegt, vorzugsweise 0,5 Gew.-% bis 18 Gew.-%.

Europäisches Patentamt

# EUROPÄISCHER RECHERCHENBERICHT

Nummer der Anmeldung
EP 04 00 8692

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | WO 02/102348 A (SIRCHI MAURIZIO ; PRISCILLA S R L UNIPERSONALE (IT)) 27. Dezember 2002 (2002-12-27) * Seite 2, Zeile 10 - Zeile 18 * * Seite 6, Zeile 13 - Seite 7, Zeile 23 * * Abbildungen 1-3 * | 1-6 | A61K7/42 |
| Y | * das ganze Dokument * ----- | 7 | |
| Y | PATENT ABSTRACTS OF JAPAN Bd. 1999, Nr. 13, 30. November 1999 (1999-11-30) & JP 11 217322 A (NONOGAWA SHOJI KK), 10. August 1999 (1999-08-10) * Zusammenfassung * ----- | 7 | |
| A | WO 97/33560 A (SCHERING PLOUGH HEALTHCARE) 18. September 1997 (1997-09-18) * das ganze Dokument * ----- | 1-7 | |

RECHERCHIERTE SACHGEBIETE (Int.Cl.7)
A61K

Der vorliegende Recherchenbericht wurde für alle Patentansprüche erstellt

| Recherchenort | Abschlußdatum der Recherche | Prüfer |
|---|---|---|
| München | 5. August 2004 | Ruckebusch, V |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 04 00 8692

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-08-2004

| Im Recherchenbericht angeführtes Patentdokument | Datum der Veröffentlichung | Mitglied(er) der Patentfamilie | Datum der Veröffentlichung |
|---|---|---|---|
| WO 02102348 A | 27-12-2002 | WO 02102348 A1 | 27-12-2002 |
| JP 11217322 A | 10-08-1999 | KEINE | |
| WO 9733560 A | 18-09-1997 | AT 239443 T | 15-05-2003 |
| | | AU 723825 B2 | 07-09-2000 |
| | | AU 1985797 A | 01-10-1997 |
| | | CA 2248876 A1 | 18-09-1997 |
| | | DE 69721780 D1 | 12-06-2003 |
| | | DE 69721780 T2 | 25-03-2004 |
| | | EP 0914083 A1 | 12-05-1999 |
| | | ES 2199349 T3 | 16-02-2004 |
| | | JP 11506130 T | 02-06-1999 |
| | | JP 2003176216 A | 24-06-2003 |
| | | WO 9733560 A1 | 18-09-1997 |

EPO FORM P0461

Für nähere Einzelheiten zu diesem Anhang : siehe Amtsblatt des Europäischen Patentamts, Nr.12/82